# EUROPEAN PATENT APPLICATION

(11) **EP 4 628 006 A1**
(43) Date of publication of application: **08.10.2025**
(21) Application number: 24738685.7
(22) Date of filing: 03.01.2024
(51) Int. Cl.: A61B 5/024, A61B 5/00, A61B 5/026

(54) **OPTICAL-BASED SKIN SENSOR, WEARABLE DEVICE COMPRISING OPTICAL-BASED SKIN SENSOR, AND OPTICAL-BASED SKIN SENSING METHOD USING SAME**

(30) Priority: 06.01.2023 KR 20230002402; 06.01.2023 KR 20230002527
(71) Applicant: SOLUM CO., LTD., Gyeonggi-do 16914 (KR)
(72) Inventor: YOON, Jung Hee, Yongin-si, Gyeonggi-do 16914 (KR)
(74) Representative: Isarpatent
(86) International application number: PCT/KR2024/000072
(87) International publication number: WO 2024/147614

(57) **Abstract**

The optical-based skin detection sensor according to an example of the present invention, comprises a first light emitting unit that outputs first output light of a first wavelength range; a second light emitting unit that outputs second output light of a second wavelength range different from the first wavelength range; a light receiving unit that senses first reflected light and second reflected light reflected, respectively, from a predetermined object in which the first output light and the second output light are detected within a predetermined distance; and a controller that determines whether the predetermined object is skin based on reflected light data for the first reflected light and the second reflected light sensed in the light receiving unit.

## Description

### [TECHNICAL FIELD]

The present invention relates to an optical-based skin detection sensor, a wearable device comprising the optical-based skin detection sensor, and an optical-based skin detection method using thereof. More specifically, it relates to an optical-based skin detection sensor that determines whether a detected object is skin based on reflected light output and reflected from a plurality of light emitting units, a wearable device comprising the optical-based skin detection sensor, and an optical-based skin detection method using thereof.

### [BACKGROUND ART]

In wearable devices measuring heart rate and the like, contact with skin is very critical to effectively irradiate generated energy into the human body. If energy is irradiated without proper contact with the skin, not only may the energy fail to be irradiated into the skin, but also adverse effects such as skin burns and the like may occur.

In order to prevent such problems, wearable devices need a mechanism for detecting contact with skin, and conventional methods for detecting skin include a method by current flow between electrodes, a method for applying a microcurrent to the human body, a method for measuring changes in impedance of an internal circuit, a method for detecting minute pressure applied to the skin, and the like.

However, according to the conventional methods, even if an object other than a human body is in contact, the contact area is recognized as a human body, and thus, problems such as battery discharge due to continuous activation and the like occur.

In addition, under unstable wearing situations in which a lot of movement is present, a large amount of noise is generated, which may cause cases where normal detection operation is not performed, and thus, inconvenience that the devices can be worn only under stable situations with minute movement or a stationary state is caused.

Furthermore, taking the recently spotlighted hearable devices (a compound word of Hear and Wearable, referring to wearable devices focused on auditory functions) as an example, since they are significantly smaller than the most generalized wearable devices, such as smart watches or VR devices, and necessarily comprise an audio output device, there are limitations to additionally equipping them with a highly sophisticated sensor solely for skin detection, and thus, there is an urgent need for an invention to solve this problem.

### [DISCLOSURE]

### [TECHNICAL PROBLEM]

The present invention has been devised to solve the problems of the conventional technology, and an object is to provide an optical-based skin detection sensor that detects skin based on reflected light which changes according to a user's blood flow, a wearable device comprising the optical-based skin detection sensor, and an optical-based skin detection method using thereof.

In addition, the present invention is to provide an optical-based skin detection sensor that detects skin based on different reflectance depending on the type of object to be detected, a wearable device comprising the optical-based skin detection sensor, and an optical-based skin detection method using the same.

Furthermore, the present invention is to provide an optical-based skin detection sensor that detect skin normally even under unstable wearing situations, a wearable device comprising the optical-based skin detection sensor, and an optical-based skin detection method using the same.

However, the technical problems to be solved by the present invention and examples of the present invention are not limited to the technical problems described above, and other technical problems may exist.

### [TECHNICAL SOLUTION]

The optical-based skin detection sensor according to an embodiment of the present invention comprises a first light emitting unit that outputs first output light of a first wavelength range; a second light emitting unit that outputs second output light of a second wavelength range different from the first wavelength range; a light receiving unit that senses first reflected light and second reflected light reflected, respectively, from a predetermined object in which the first output light and the second output light are detected within a predetermined distance; and a controller that determines whether the predetermined object is skin based on reflected light data for the first reflected light and the second reflected light sensed in the light receiving unit.

In addition, the first output light is red light with a wavelength band of 400nm to 700nm, and the second output light is infrared (IR) light with a wavelength of 850nm to 1050nm.

Furthermore, the controller, determines whether the predetermined object is skin in a stationary state based on the first reflected light and the second reflected light.

Moreover, the controller determines that the object is skin if a ratio of a normalized value of an intensity of the first reflected light and a normalized value of an intensity of the second reflected light falls within a predetermined criterion.

In addition, the controller, determines whether the predetermined object is skin in a moving state based on the first reflected light and the second reflected light.

Furthermore, the controller, determines that the object is skin if a ratio of a normalized value of a rate of change of intensity of the first reflected light and a normalized value of a rate of change of intensity of the second reflected light falls within a predetermined criterion.

In addition, the controller, controls the second light emitting unit in a non-wearing state to control it to output the second output light at a first output strength, and detects a predetermined object within a predetermined distance based on the second reflected light for the second output light in the light receiving unit.

Moreover, the controller, controls the first light emitting unit to output the first output light, when a predetermined object is detected within the predetermined distance.

In addition, the reflected light data is data which consists of a combination of a signal by blood flow, P(t) and a signal by motion, M(t), and includes signal patterns of the first and second reflected light.

Furthermore, the controller, filters only the P(t) by removing the M(t) included in the reflected light data based on a blind separation technique, when the reflected light data includes the M(t) comprising a DC component equal to or greater than a predetermined criterion.

In addition, the blind separation technique, includes a PCA (Principal Component Analysis) method that minimizes cross-correlation of the reflected light data and an ICA (Independent Component Analysis) method that minimizes mutual information.

On the other hand, the wearable device according to an embodiment of the present invention, comprises a skin detection sensor comprising; a first light emitting unit that outputs first output light of a first wavelength range; a second light emitting unit that outputs second output light of a second wavelength range different from the first wavelength range; a light receiving unit that senses first reflected light and second reflected light reflected, respectively, from a predetermined object in which the first output light and the second output light are detected within a predetermined distance; and a controller that determines whether the predetermined object is skin based on reflected light data for the first reflected light and the second reflected light sensed in the light receiving unit, wherein a processor of the wearable device that controls the wearable device, determines that it is in a wearing state upon determining that the type of the predetermined object is skin, and controls activation of the wearable device based on the determined wearing state.

In addition, the optical-based skin detection method according to an embodiment of the present invention is a method of detecting optical-based skin by at least one processor of a wearable device, and comprises; outputting first output light of a first wavelength range based on a first light emitting unit; outputting second output light of a second wavelength range different from the first wavelength range based on a second light emitting unit; sensing first reflected light and second reflected light reflected, respectively, from a predetermined object in which the first output light and the second output light are detected within a predetermined distance based on a light receiving unit; determining whether the predetermined object is skin based on reflected light data for the sensed first reflected light and the second reflected light; determining that it is in a wearing state upon determining that the type of the predetermined object is skin; and controlling activation of the wearable device based on the determined wearing state.

Furthermore, the determining whether the predetermined object is skin, comprises determining whether the predetermined object is skin in a stationary state based on the first reflected light and the second reflected light, and the determining whether the predetermining object is skin in a stationary state, comprises determining that the object is skin if a ratio of a normalized value of an intensity of the first reflected light and a normalized value of an intensity of the second reflected light falls within a predetermined criterion.

In addition, the determining whether the predetermined object is skin, comprises determining whether the predetermined object is skin in a moving state based on the first reflected light and the second reflected light, and the determining whether the predetermining object is skin in a moving state, comprises determining that the object is skin if a ratio of a normalized value of a rate of change of intensity of the first reflected light and a normalized value of a rate of change of intensity of the second reflected light falls within a predetermined criterion.

### [ADVANTAGEOUS EFFECTS]

The optical-based skin detection sensor, wearable device comprising the optical-based skin detection sensor, and the optical-based skin detection method using the same according to the embodiments of the present invention, have an effect of increasing user satisfaction by quickly and accurately identifying a wearing state even under unstable wearing situations, thereby eliminating unnecessary battery waste, and at the same time, providing various services such as exercise coaching services, sleep pattern analysis services, and the like.

In addition, the optical-based skin detection sensor, wearable device comprising the optical-based skin detection sensor, and the optical-based skin detection method using the same according to the examples of the present invention, have an effect of increasing efficiency in using wearable devices by detecting skin based on different reflectance according to the type of object to be detected, thereby rapidly determining the wearing state.

Furthermore, the optical-based skin detection sensor, wearable device comprising the optical-based skin detection sensor, and the optical-based skin detection method using the same according to the examples of the present invention, have an effect of increasing usability by detecting skin normally even under unstable wearing situations, thereby allowing the corresponding wearable device to be worn conveniently regardless of the situation in any time.

However, the effects to be obtained by the present invention are not limited to the effects described above, and other effects not mentioned can be clearly understood from the following description.

### [BRIEF DESCRIPTION OF THE DRAWINGS]

FIG. 1 is an internal block diagram of a skin detection sensor according to an embodiment of the present invention.
FIG. 2 is a cross-sectional view to illustrate the structure of a skin detection sensor according to an embodiment of the present invention.
FIG. 3 is an example showing the structure of an optical filter according to an embodiment of the present invention.
FIG. 4 is a flowchart to illustrate a method by which a skin detection sensor according to an embodiment of the present invention determines whether a user is wearing.
FIG. 5 is an example of a graph showing changes in optical reflection signals based on movement according to an embodiment of the present invention.
FIG. 6 is an example of a table showing wavelength band reflection rates by type of object according to an embodiment of the present invention.
FIG. 7 is an example of a graph showing changes in wavelength band reflection rates by blood flow according to an embodiment of the present invention.

### [MODE FOR INVENTION]

The present invention can apply various modifications and may have a variety of embodiments, and thus, specific embodiments are illustrated in the drawings and described in the detailed description in detail. The effects and features of the present invention, and methods for achieving them, will become apparent with reference to embodiments described in detail below with drawings. However, the present invention is not limited to the embodiments disclosed below, but may be implemented in various forms. In the following embodiments, the terms such as first, second, and the like are not limitative meanings, but they are used merely to distinguish one component from another component. In addition, singular expressions include plural expressions, unless clearly indicated otherwise by context. Furthermore, terms such as comprise or have or the like mean the presence of features or components described in the specification, and are not intended to exclude the possibility that one or more other features or components are added in advance. In addition, in the drawings, for convenience of explanation, the size of the components may be exaggerated or reduced. For example, since the size and thickness of each component shown in the drawings are arbitrarily represented for convenience of explanation, the present invention is not necessarily limited to what is illustrated.

Hereinafter, the embodiments of the present invention will be described in detail with reference to the accompanying drawings, and when describing with reference to the drawings, identical or corresponding components are denoted by the same reference numbers and redundant descriptions thereof will be omitted.

### Skin Detection Sensor (100)

The skin detection sensor (100) according to an embodiment of the present invention may be a predetermined complex sensor mounted in a predetermined wearable device (for example, smart watch and/or wireless earphones).

Then, the wearable device according to an embodiment of the present invention, is described with reference to wireless earphones implemented as a canal type TWS, but it may be implemented in any device as long as it is a device that can be worn on a user's body.

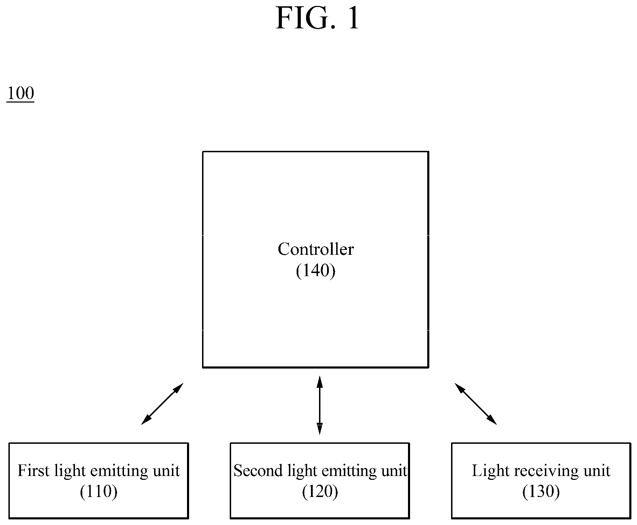

FIG. 1 is an internal block diagram of a skin detection sensor according to an embodiment of the present invention.

The skin detection sensor (100) according to the embodiment, may comprise at least one component of a first light emitting unit (110), a second light emitting unit (12), a light receiving unit (130) and a controller (140).

The first light emitting unit (110) and the second light emitting unit (120), may output light of wavelength bands different from each other.

In detail, a light emitting device of the first light emitting unit (110), may comprise a first light emitting device that outputs light of a first wavelength range.

Then, the first light emitting unit (110), may be Visual RGB color LED (VCSEL) which generates light of a wavelength of about 400 nm ~ 700 nm. Hereinafter, for convenience of explanation, the light generated by the first light emitting unit (110) will be described with reference to RED light.

In addition, the second light emitting unit (120), may comprise a second light emitting device that outputs light of a second wavelength range.

Then, the light emitting device of the second light emitting unit (120), may be infrared rays (NIR) LED (VCSEL) which generates light of a wavelength of about 850nm ~ 1050nm.

In addition, the light receiving unit (130), may sense scattered or reflected light.

Furthermore, the light receiving unit (130), may comprise an optic module equipped with a photo diode, a photo transistor and a photo detector.

In detail, the light receiving unit (130), may comprise a photo detector which senses reflected light of a first wavelength range outputted from the first light emitting unit (110), and reflected light of a second wavelength range outputted from the second light emitting unit (120).

This light receiving unit (130), may comprise a light sensor that senses reflected light and an optical filter (3110) coated on the light sensor in the embodiment.

Then, the light receiving unit (130), may be implemented as two light receiving units corresponding to the number of the first light emitting unit (110) and the second light emitting unit (120), or be implemented as one light receiving unit that detects light of the first light emitting unit (110) and the second light emitting unit (120) at the same time. Hereinafter, for convenience of explanation, it will be described with reference to one implemented as one light receiving unit.

The controller (140) may comprise an integrated driver that controls the first light emitting unit (110) and the second light emitting unit (120).

In addition, the controller (140), may comprise an integrated controller that collects and adjusts analog data sensed from the photo detector of the light receiving unit (130).

Furthermore, the controller (140), may generate adjacent data (as an embodiment, data on the type of an adjacent object) based on the sensed analog data.

In addition, the controller (140), may determine whether a user is wearing the wearable device based on the generated adjacent data.

In another embodiment, the controller (140) may generate analog data sensed from the photo detector as adjacent data, transfer the generated adjacent data to a main processor of the wearable device equipped with the skin detection sensor, and determine whether the wearable device is worn based on the adjacent data of the main processor.

Hereinafter, it will be described as the controller (140) comprises the main processor.

In addition, the controller (140) may comprise an ROIC (Read Out IC) that converts light information obtained based on the first and second light emitting units (110, 120) and the light receiving unit (130) into electrical information, and processes it by removing noise and the like.

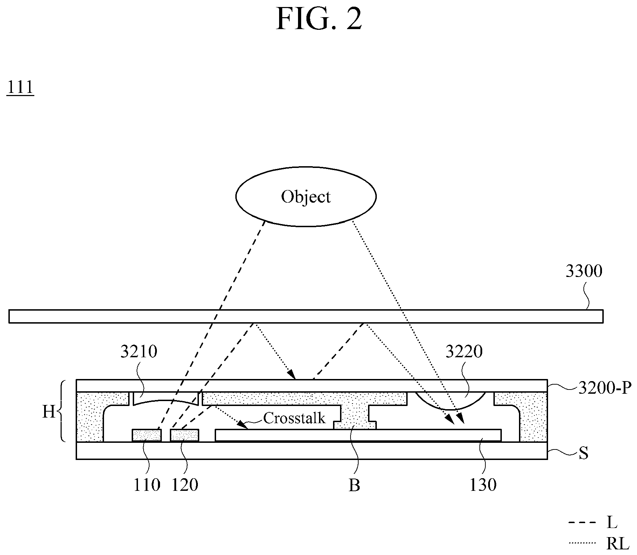

FIG. 2 is a cross-sectional view to illustrate the structure of a skin detection sensor according to an embodiment of the present invention.

Referring to FIG. 2, in the embodiment, the skin detection sensor (100), may comprise a substrate (S), a housing (H), a diaphragm (B) and optical units (3200-P, 3210, 3220, 3300). In addition, the optical unit may comprise an optical plate (3200-P), a first lens (3210), a second lens (3220) and a glass unit (3300).

It may comprise a substrate (S) which supports the light receiving unit (130) disposed next to the first light emitting unit (110) and the second light emitting unit (120).

In addition, in the embodiment, the skin detection sensor (100), may comprise a housing (H) which receives the first light emitting unit (110), the second light emitting unit (120) and the light receiving unit (130), and is disposed on the substrate.

Furthermore, in the embodiment, the housing (H), may comprise a diaphragm (B) disposed between the first and second light emitting units (110, 12) and the light receiving unit (130).

The diaphragm (B) may be positioned to prevent light (L) emitted from the first and second light emitting units (110, 120) from being reflected inside the housing (H) rather than blood vessels of a user's body and detected by the light receiving unit (130).

In addition, in the embodiment, the skin detection sensor (100), may further comprise an optical plate (3200-P) comprising a first lens (3210) that diffuses light (L) outputted from the first and second light emitting units (110, 120), and a second lens (3220) that refracts reflected light (RL) toward the light receiving unit (130).

In detail, the first lens (3210), may be formed and positioned on the location spaced apart from the first and second light emitting units (110, 120), and the second lens (3220), may be formed and positioned on the location spaced apart from the light receiving unit (130).

On the other hand, in the embodiment, the light emitting unit (130), may comprise an optical filter with a predetermined structure to transmit only reflected light (RL) incident on a predetermined location in the light receiving unit (130).

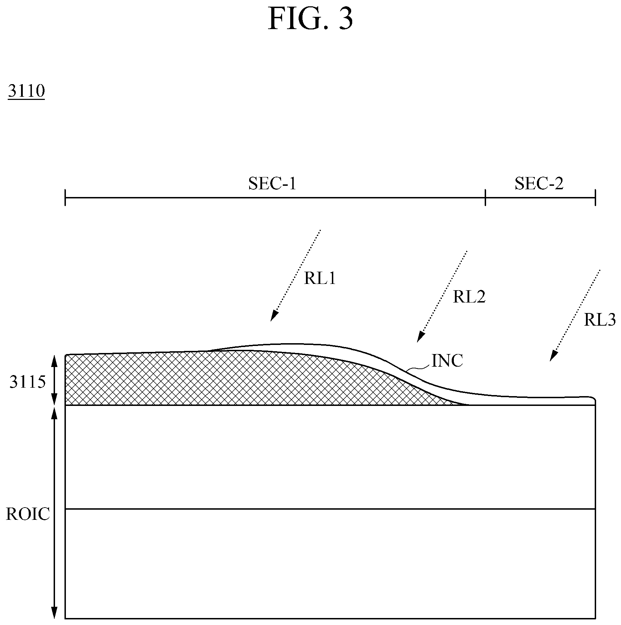

FIG. 3 is an example showing the structure of an optical filter according to an embodiment of the present invention.

Referring to FIG. 3, in the embodiment, the optical filter (3110) comprised in the light receiving unit (130), may comprise an IR filter (3115) equipped with an inclined surface (INC) with a predetermined incline.

Herein, the IR filter (3115) according to the embodiment, may be an infrared filter, which is an infrared pass filter. This IR filter (3115) may have a structure that transmit the visible light range of incident light and reflects the near-infrared range.

In addition, in the embodiment, the optical filter (3110) comprising the IR filter (3115), may be divided into a first section (SEC-1) that can receive predetermined reflected light (RL) and a second section (SEC-2) that cannot receive the reflected light (RL), based on the inclined surface (INC).

Then, the first and second light (RL1, RL2) entering the first section (SEC-1) and inclined surface (INC), can be transmitted through the IR filter (3115) disposed at the lower part of the first section (SEC-1) and inclined surface (INC).

However, the third right (RL3) entering the second section (SEC-2), may not be transmitted, as the IR filter (3115) disposed at the lower part is not present.

The optical filter (3110) having the afore-mentioned structure, may not receive the corresponding light primarily, as the IR filter (3115) is not comprised at the lower part of light incident on an abnormal location.

Accordingly, in the embodiment, the skin detection sensor (100), may filter and receive at least one reflected light based on the optical filter (3110).

Hereinafter, operations performed by the integrated driver, integrated controller and ROIC of the skin detection sensor (100) may be described as being performed by the skin detection sensor (100) (specifically, the controller (140) of the skin detection sensor (110)).

This skin detection sensor (100), may determine whether a user is wearing by determining whether an object is skin detected based on the reflected light outputted and reflected from the first and second light emitting units (110, 120).

### Method for determining whether a user is wearing by the skin detection sensor

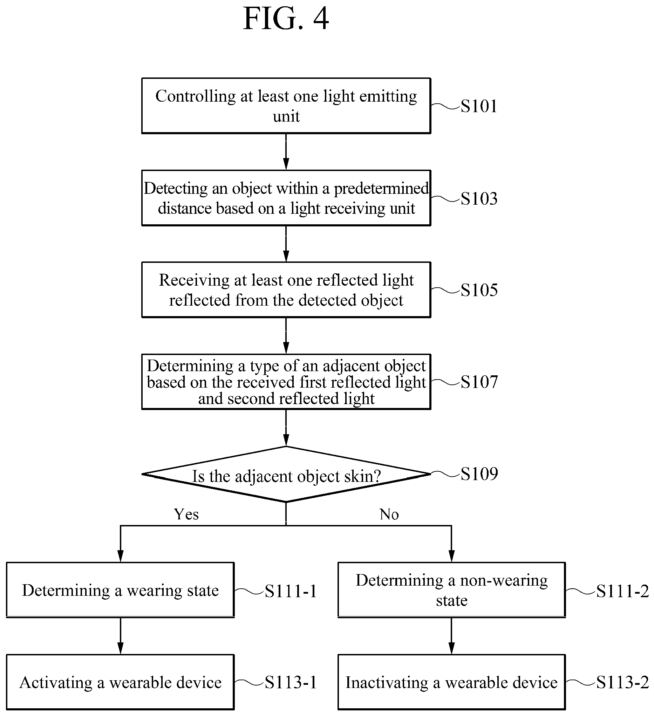

Hereinafter, a method for determining whether a user is wearing by the controller (140) of the skin detection sensor (100) according to an embodiment of the present invention will be described in detail with reference to the attached FIG. 4 to FIG. 7.

FIG. 4 is a flowchart to illustrate a method by which a skin detection sensor according to an embodiment of the present invention determines whether a user is wearing.

Referring to FIG. 4, in the embodiment, the controller (140), may control at least one light emitting unit (110, 120). (S101)

In detail, in the embodiment, the controller (140), may control at least one light emitting unit of the first light emitting unit (110) and/or the second light emitting unit (120) in a non-wearing state.

More specifically, in the embodiment, the controller (140), may control the first light emitting unit (110) and/or the second light emitting unit (120) so as to output predetermined light by operating at least one light emitting device among the first light emitting device that outputs light of the first wavelength range of the first light emitting unit (110) and/or the second light emitting device that outputs light of the second wavelength range of the second light emitting unit (120).

Accordingly, in the embodiment, the first and second light emitting units (110, 120) of the controller (140), may output at least one light among the first light of the first wavelength range and/or the second light of the second wavelength range.

Then, in the embodiment, the first light may be RED light, and the second light may be IR (infrared rays).

Since the RED light is visible and consumes high power for the light output, whereas the IR light (infrared rays) is invisible and consumes low power for the light output, in the embodiment, it may be preferable to control only the second light emitting unit (120) that outputs the second light.

In addition, the controller (140), may sense bio data (e.g., PPG) through the first light emitting unit (110) and the second light emitting unit (120) in a wearing state, and in order to sense precise bio data, the light emitting unit may be controlled at a first output strength to output light.

Furthermore, the controller (140), may control only the second light emitting unit (120) to output the second light with a second output strength relatively lower than the first output strength, in order to detect proximity with lower power in a non-wearing state.

In addition, in the embodiment, the controller (140), may detect an object within a predetermined distance based on reflected light of light outputted from the light emitting unit. (S103)

In detail, in the embodiment, the controller (140), may output infrared rays (as the embodiment, the second light) into an adjacent object using a first principle that is a principle of an optical distance sensor, and detect the infrared rays reflected from the adjacent object and returning by the light receiving unit, and detect the object within the predetermined distance, thereby determining proximity.

Then, the distance between the predetermined skin detection sensor which serves as the criterion for proximity and the adjacent object may be, for example, approximately 20cm.

In other words, in the embodiment, the controller (140), may detect the object within the predetermined distance based on time taken to receive reflected light, and determine that there is an object within the predetermined distance when the intensity of the reflected light is greater than a predetermined value.

However, the method for detecting an object using the first principle, may cause problems that there is a high probability of error in detecting an object within a predetermined distance, and an object other than a person is detected as skin depending on proximity, as an error range in the distance at which an object can be detected is large, and the degree of intensity of infrared rays reflected from the object and returning may be different depending on the type of object.

Accordingly, in the embodiment, the controller (140), may additionally control the light emitting unit, in order to distinguish whether the object is skin.

In other words, in the embodiment, the controller (140), may more accurately distinguish the type of the adjacent object based on a plurality of the detected reflected light, or more accurately determine whether a user is wearing even in a situation where movement is present, by detecting a plurality of reflected light outputted from a plurality of light emitting units, respectively.

In addition, in the embodiment, the controller (140), may receive a plurality of reflected light reflected from the detected object. (S105)

In detail, in the embodiment, the controller (140), may receive at least one reflected light filtered based on the optical filter of the light receiving unit among the reflected light reflected from the detected object. Then, in the embodiment, the reflected light received, will be described with reference to the first reflected light which is the first light reflected and the second reflected light which is the second light reflected.

Referring to FIG. 3 again, in the embodiment, the controller (140), may detect the first and second reflected light (RL1, RL2) incident at a normal location based on the optical filter (3110) of the light receiving unit (130).

In the embodiment, the controller (140), may transmit only the first and second reflected light (RL1, RL2) entering the first section (SEC-1) and inclined surface (INC) through the IR filter (3115).

Accordingly, in the embodiment, the controller (140), may filter and receive at least one reflected light (as the embodiment, the first and second reflected light) based on the optical filter (3110).

In addition, in the embodiment, the controller (140), may receive reflected light data including signal patterns of the filtered first and second reflected light.

Furthermore, in the embodiment, the controller (140), may determine the type of the adjacent object based on the received at least one reflected light. (S107)

In detail, in the embodiment, the controller (140), may determine the type of the adjacent object based on 1) the size of AC components, 2) wavelength band reflection rates by type of the object, and/or 3) wavelength band reflection rates by blood flow, in the received at least one reflected light (in the embodiment, the first reflected light and/or the second reflected light).

Then, the reflected light according to the embodiment, may be received in different patterns according to whether the detected object is skin and/or whether movement is present.

Herein, the movement, may mean movement of the controller (140) according to the embodiment and/or the wearable device comprising the controller (140) itself, not movement of the detected object.

In addition, a predetermined signal (S(t)) comprising reflected light according to the embodiment, may be composed of environmental influence (E(t)), PPG influence (P(t)), movement influence (M(t)) and/or other noise (N(t)). Accordingly, the equation of S(t) = E(t) + P(t) + M(t) + N(t) may be derived.

Then, assuming that the environmental influence, E(t) is a constant, and the other noise, N(t) is negligible, and that the AC components of P(t) and M(t) are represented as p(t) and m(t), the AC component signal, s(t) may be expressed as the equation of s(t) = p(t) + m(t).

In other words, according to the multiple equations derived above, factors affecting the AC component signal may be PPG (photoplethysmography) and movement.

Herein, PPG may refer to a blood flow volume in blood vessels by irradiating light into a predetermined object and measuring a user's pulse and the like using light transmitted or reflected therefrom.

In other words, as this PPG (photoplethysmography) is data measurable only in skin, the factors affecting the AC component signal may be whether the detected object is skin and/or whether movement is present.

Therefore, depending on the case, in the embodiment, the reflected light, may be received in different patterns in a total of 4 states which are a state in contact with an inanimate object and stationary, a state in contact with an inanimate object and in motion, a state in contact with skin (wearing) and stationary, and a state in contact with skin (wearing) and in motion.

Then, in the embodiment, the inanimate object, may refer to an object not skin.

In addition, the controller (140) may determine whether an adjacent object is skin or an inanimate object even in a state in motion or not in motion, depending on each pattern of these 4 kinds of states.

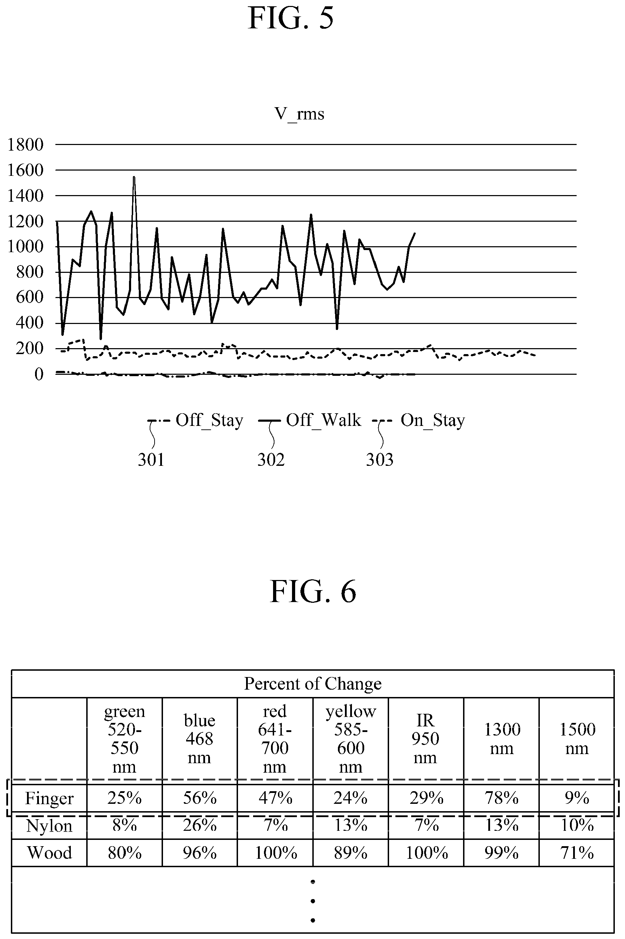

FIG. 5 is an example of a graph showing changes in optical reflection signals based on movement according to an embodiment of the present invention. For convenience of explanation, it may be assumed that the optical reflection signal is a signal for the second reflected light, which is infrared.

Referring to FIG. 5, in the embodiment, the controller (140), may determine the type of the adjacent object based on 1) the size of AC components.

In the embodiment, the controller (140), may receive a first signal (301) which is an optical reflection signal comprising an AC component (for example, 0 to 10) with a very small fluctuation range, in the state in contact with an inanimate object and stationary.

This is because, assuming that no noise occurs due to movement, if there is no skin, or an inanimate, in contact with the controller (140), no change in reflection rate by blood flow occurs, so the reflection rate is constantly maintained and the AC component is very small.

In addition, in the embodiment, the controller (140), may receive a second signal (302), which is an optical reflection signal including an AC component (for example, 200 to 1800) with a very large fluctuation range, in the state in contact with an inanimate object and in motion.

This is because, regardless of whether the object in contact with the controller (140) is skin or an inanimate object, when noise by movement occurs, reflected light cannot be sensed continuously at the same part, so the intensity and reflection rate of the first and second reflected light are irregularly detected, and therefore, the AC component becomes very larger.

Furthermore, in the embodiment, the controller (140), may receive a third signal (303) which is an optical reflection signal including an AC component (for example, 150 to 200) with a small and constant fluctuation range, in the state in contact with skin and stationary.

This is because, the reflected light is continuously sensed from the same part of the skin in contact with the controller (140) and has a constant reflection rate (for example, 29% in case of infrared rays for a human finger), so the AC component is small and constant.

In addition, the third signal (303) may comprise a predetermined section in which the AC component is measured relatively large at a predetermined time interval by blood flow within the skin. The predetermined section may be a section in which a user's pulse is measured.

In other words, in the embodiment, the controller (140), may determine the type of the adjacent object as an inanimate object, when the received at least one reflected light includes the size of the AC component similar to the first signal (310).

However, in the embodiment, determining the type of the adjacent object only based on the size of the AC component of one reflected light, may have very low accuracy and efficiency in that the amount of light (intensity) of the received optical reflection signal is changed by changes in movement, or the refection rate is changed by changes in blood flow, except for the state in contact with an inanimate object and stationary.

Accordingly, in the embodiment, the controller (140), may determine the type of the adjacent object based on 2) the reflection rate of reflected light by wavelength band depending on the type of object using a plurality of reflected light, not one reflected light.

FIG. 6 is an example of a table showing wavelength band reflection rates by type of object according to an embodiment of the present invention.

Referring to FIG. 6, the reflected light according to the embodiment, the reflection rate by wavelength band depending on the type of object may have a specific value.

For example, when the reflected light is in contact with a finger, the reflection rate with specific values of 47% for the reflection rate of Red light, and 29% for the reflection rate of IR light, may be derived.

Then, at a distance where the reflected light is irradiated to the object, due to differences in environmental changes such as distance, the signal may change due to changes in the amount of light. However, the difference in the ratio of the reflection rate of the reflected light of different wavelength bands may be always maintained at a level at which the ratio of the reflection rate of the infrared light compared to the RED light is about 1.5 times greater, because the environment is the same.

In the embodiment, assuming that the reflected signal is denoted by S, and the light source size is denoted by L, and the reflection rate is denoted by r, it may be S = r L.

Furthermore, in the embodiment, as the effect by movement changes only the substantial light source size, assuming that the change in the signal resulting when the size of the light source is changed from L_0 to L_1 by movement is S_0 and S_1, the equations of S_0 = r L_0 and S_1 = r L_1 may be derived.

In addition, assuming L_1 = a L_0, the difference in the signal size may be derived as the equation of Delta S = S_1 - S_0 = r (L_1 - L_0) = (a-1) r L_0.

If this, is normalized to the basic signal size S_0, (Delta S)_nor = Delta S / S_0 = a-1 is derived, so in other words, the change in the normalized signal size may be a value related only to the change in the size of the light source which is not related to the reflection rate or the size of the light source.

Therefore, since (Delta S^IR)_nor / (Delta S^red)_nor = 1, it can be seen that the ratio of the rate of change of the first reflected light and the second reflected light according to the change in distance is the same (synchronized) even if there is m(t) (effect of movement).

For example, when 100 is irradiated, if 70 is derived, as the reflection rate is 70%, the distance at which the reflected light is irradiated is far, so 63 may be derived when 90 is irradiated.

Herein, assuming that the reflection rate of IR light is 30% and the reflection rate of Red light is 50%, when light of 100 each is irradiated, the value of 30, 50 may be derived, respectively, and as the distance becomes far, when light of 90 is irradiated, the value of 27, 45 may be derived, respectively.

Thus, as the ratio of the reflection rate is constant as 3:5 all for 30:50 and 27:45, it can be seen that the ratio of the rate of change of the first reflected light and the second reflected light according to the change in distance is the same.

In other words, in the embodiment, the controller (140), may determine that the type of the adjacent object is skin, if the ratio of the rate of change of the received first reflected light and the second reflected light is within a predetermined value and a certain range from the corresponding value.

However, there may be cases where noise occurs in a situation where movement exists, and the like, and reflected light with an abnormal reflection rate is sensed. Therefore, in the embodiment, the method in which the controller (140) determines the type of the adjacent object based on only the ratio of the reflection rate of the wavelength band by object type has the disadvantage of low accuracy and efficiency.

In order to solve the disadvantage, in the embodiment, the controller (140) may determine the type of the adjacent object based on 3) the wavelength band reflection rate by PPG (blood flow) of the normalized first reflected light and second reflected light. In another aspect, in the embodiment, the controller (140), may determine the type of the adjacent object based on the ratio of the reflection rate of a plurality of reflected light after removing noise from the reflected light of 2 wavelength bands.

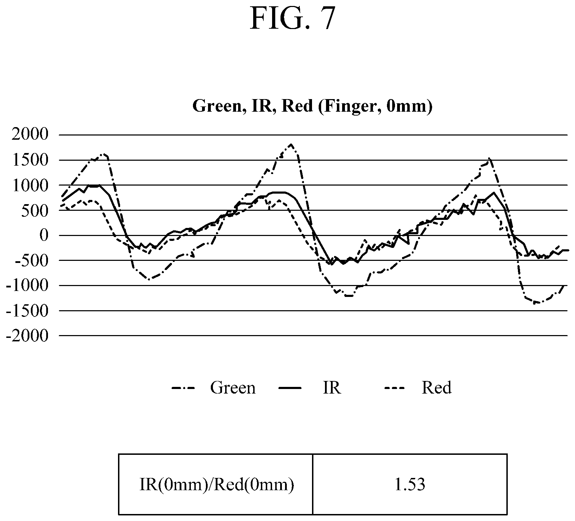

FIG. 7 is an example of a graph showing changes in wavelength band reflection rates by blood flow according to an embodiment of the present invention.

Referring to FIG. 7, in the embodiment, the reflected light, may exhibit changes in wavelength band reflection rate depending on changes in blood flow measured on a user's skin.

In detail, in the embodiment, the reflected light, may exhibit changes in wavelength band reflection rate as the amount of Hb and Hb02 changes depending on changes in blood flow in capillary vessels in the contact part in a user's skin.

In other words, the method for determining the type of the adjacent object based on the wavelength band reflection rate by blood flow, may be said to be the same method as the PPG sensor principle of a predetermined wearable device.

Then, the rate of change of the reflection rate that changes according to the flow of blood, may be always the same as the ratio is synchronized, in the light of the first wavelength band and the light of the second wavelength band.

For example, when the reflected light is in contact with an inanimate object, the ratio of p_Red(t) which is the AC component of RED light and p_IR(t) which is the AC component of IR light may be derived as 1 and within a certain range.

For example, when the reflected light is in contact with skin, the ratio of p_Red(t) which is the AC component of RED light and p_IR(t) which is the AC component of IR light may be derived as 1.5 and within a certain range.

In the embodiment, the signal measured on skin may be represented by the equation of S(t) = ( r_blood V(t) + r_other ) L.

Herein, r_blood is a reflection rate by blood, and the corresponding signal may change in the form of r_blood V(t) depending on the change in blood flow V(t).

In addition, r_other is a reflection rate by human body tissue such as skin, bones, and the like, which does not change and is fixed, and it may be a constant.

Furthermore, the light source L may be a fixed value under assumption that movement is constant.

Then, in the embodiment, when the controller (140) comes into contact with skin and the blood flow V(t) changes from V_0 to V_1, the difference in signals
may be derived as the equation of Delta S = ( r_blood V_1 + r_other ) L - ( r_blood V_0 + r_other ) L = r_blood ( V_1 - V_0 ) L.

Furthermore, when the Delta S is normalized, it may be derived as the equation of (Delta S)_nor = { r_blood (V_1 - V_0) L } / { (r_blood V_ave + r_other) L } = r_blood (V_1 - V _0)/(r_blood V_ave + r_other).

In addition, assuming that r_other is greater than r_blood V_ave, it may be (Delta S)_nor = r_blood (V_1 - V_0) / r_other.

Herein, when the ratio of the IR light and Red light is calculated, it may be derived as the equation of (Delta_S^IR)_nor / (Delta S^red)_nor = {r_blood^IR (V_1 - V_0) / r_other^IR} / {r_blood^red (V_1 - V_0) / r_other^red} = {r_blood^IR / r_other^IR} / {r_blood^red / r_other^red}.

In summary, in the embodiment, the derived equation may always have a constant value under measurement situations regardless of the presence of movement, and the ratio of the change in the reflection rate of the first reflected light and the second reflected light upon skin contact may be always maintained at a value of 1.5.

In other words, in the embodiment, the controller (140), may normalize reflected light data including the first reflected light and the second reflected light, and determine the type of the detected object according to the normalized numerical value.

In detail, in the embodiment, the controller (140), may determine that the type of the detected object is an inanimate object, when the ratio of the rate of change is 1 (and within a certain range), and the type of the detected object is skin, when the ratio of the rate of change is 1.5 (and within a certain range), in the reflection rate of the second reflected light compared to the first reflected light during normalization.

This may be because, in skin, the first reflected light, RED light changes more than the second reflected light, IR light, resulting in a higher rate of change, and in a non-skin object, the intensity and rate of change of the first reflected light and the second reflected light are same.

In addition, the controller (140) according to the embodiment, may obtain data excluding m(t) (effect of movement) from the reflected light data by removing a DC value.

For this, in the embodiment, the controller (140), may use a blind separation technique for separating the two signals, when the changes in signals by blood flow and changes in signals by movement are mixed.

The blind separation technique may be a technique for separating the P(t) and M(t) using the fact that the received signals (as an embodiment, the first and second reflected light) consist of a combination of P(t) (signals by blood flow) and M(t) (signals by movement) that are not correlated with each other.

Then, the blind separation technique, may include a PCA (Principal Component Analysis) method that minimizes cross-correlation of two signals and an ICA (Independent Component Analysis) method that minimizes mutual information.

Accordingly, in the embodiment, the controller (140) may remove M(t) from the reflected light and estimate the signals by blood flow based on the blind separation method.

In other words, in the embodiment, the controller (140), may remove DC components from a plurality of reflected signals measured under measurement situations where movement is present, and determine that the adjacent object is skin, if the ratio of the rate of change is within a certain range from the value of 1.5 in the reflection rate of the second reflected light compared to the first reflected light which are normalized.

Furthermore, in the embodiment, the controller (140), may determine whether the determined type of the adjacent object is skin. (S109)

In summary, in the embodiment, the controller (140), may perform normalization regardless of presence of movement.

In addition, in the embodiment, the controller (140), may remove DC components comprised in the normalized first and second reflected light data.

Furthermore, in the embodiment, the controller (140), may determine that the adjacent object is an inanimate object not skin, if the ratio of the rate of change is derived as 1 and within a predetermined certain range from 1, in the reflection rate of the second reflected light compared to the first reflected light under normalization and DC components-removed conditions.

In addition, in the embodiment, the controller (140), may determine that the adjacent object is skin, if the ratio of the rate of change is derived as 1.5 and within a predetermined certain range from 1.5, in the reflection rate of the second reflected light compared to the first reflected light under normalization and DC components-removed conditions.

However, in the embodiment, as the reflection rate, is measured differently depending on skin color of a person, and the like, the criterion of the reflection rate value may vary within a predetermined range.

Therefore, in the embodiment, the controller (140), may perform calibration when the wearable device comprising the skin detection sensor (100) according to the embodiment is initially worn. As a result, in the embodiment, the controller (140), may re-establish the criterion of the reflection rate depending on the reflection rate value derived according to the skin color of the user using the wearable device.

In addition, in the embodiment, the controller (140), may control activation of the wearable device depending on the result of determining the determined type of the adjacent object.

In the embodiment, the controller (140), may determine that the wearing state is wearing, if the determined type of the adjacent object is skin. (S111-1)

Then, in the embodiment, the controller (140), may re-determine whether to maintain wearing by returning to step S101 at predetermined intervals and sequentially re-executing the steps S101 to S109, if it is determined that there is an adjacent object.

When re-determining whether to maintain wearing depending on whether the adjacent object is skin, if the ratio of the rate of change of the second reflected light compared to the first reflected light is derived as a value within a certain range from 1.5 again, the wearing state may be maintained as wearing.

Furthermore, in the embodiment, the controller (140), may transmit a control signal that activates a wearable device into a linked wearable device, when the wearing state is determined as wearing. (S113-1)

On the other hand, in the embodiment, the controller (140), may determine that the wearing state is non-wearing, if the determined type of the adjacent object is an element other than skin. (S111-2)

In the same manner, in the embodiment, the controller (140), may re-determine the type of the adjacent object, by returning to step S101 at predetermined intervals and sequentially re-executing the steps S101 to S109, if the wearing state is determined as a non-wearing state.

When the reflection rate of infrared light compared to red light is normalized, depending on the re-determination of the type of the adjacent object, the wearing state may be re-determined as wearing, if a value within a certain range from 1.5 is derived, and the wearing state may be re-determined as non-wearing, if a value other than that is derived.

Furthermore, in the embodiment, the controller (140), may transmit a control signal that inactivates a wearable device into a linked wearable device, when the wearing state is determined as non-wearing. (S113-2)

In other words, in the embodiment, according to the control signal that activates the wearable device of the controller (140), the wearable device according to the embodiment may provide various services (for example, exercise coaching services, sleep pattern analysis services, and the like) that may be provided into a user based on the wearable device.

As a result, the controller (140) according to the embodiment, has an effect of drastically reducing the malfunction rate of the sensor to increase battery economy, by determining whether a user is wearing depending on the wavelength band reflection rate by blood flow based on the principle of the PPG sensor comprising a plurality of light emitting units.

As described above, the optical-based skin detection sensor according to the embodiment of the present invention, the wearable device comprising the optical-based skin detection sensor, and the optical-based skin detection method using the same, detect skin based on reflected light that changes depending on user blood flow, and thus, have an effect of increasing user satisfaction by quickly and accurately identifying a wearing state, thereby eliminating unnecessary battery waste, and at the same time, providing various services (for example, exercise coaching services, sleep pattern analysis services, and the like.

Furthermore, the optical-based skin detection sensor according to the embodiment of the present invention, the wearable device comprising the optical-based skin detection sensor, and the optical-based skin detection method using the same, detect skin based on the reflection rate different by type of the object to be detected, thereby quickly determining the wearing state, and thus, have an effect of increasing efficiency in the use of the wearable device.

In addition, the optical-based skin detection sensor according to the embodiment of the present invention, the wearable device comprising the optical-based skin detection sensor, and the optical-based skin detection method using the same, have an effect of increasing usability by detecting skin normally even under unstable wearing situations, thereby allowing the corresponding wearable device to be worn conveniently regardless of the situation in any time.

The embodiments according to the present invention described above may be implemented in the form of program instructions that can be executed through various computer components and be recorded on computer readable recording media. The computer readable recording media may include program instructions, data files, data structures and the like alone or in combination. The program instructions recorded in the computer readable recording media may be specially designed and configured for the present invention or be known and available to those skilled in the field of computer software. Examples of the computer readable recording media, include magnetic media such as hard disks, floppy disks, and magnetic tapes, optical recording media such as CD-ROM and DVD, magneto-optical media such as floptical disks, and hardware devices specially configured to store and execute program instructions, such as ROM, RAM, flash memory, and the like. Examples of the program instructions, include not only machine language codes such as those generated by a compiler, but also high-level codes that can be executed by a computer using an interpreter or the like. The hardware devices may be changed into one or more software modules to perform the process according to the present invention, and vice versa.

The specific implementations described in the present invention are presented as one or more embodiments, and are not intended to limit the scope of the present invention in any way. For brevity of the specification, descriptions of conventional electronic components, control systems, software, and other functional aspects of the systems may be omitted. In addition, connections or connection members of lines between components illustrated in the drawings illustratively show functional connections and/or physical or circuit connections, and may be redisposed, or shown as additional various functional connections, physical connections, or circuit connections in actual devices. Furthermore, unless specifically stated as "essential," "critically", or the like, they may not be necessarily required components for application of the present invention.

Furthermore, the detailed description of the present invention has been provided with reference to preferred embodiments of the present invention, but it will be understood by those skilled in the art or those having ordinary knowledge in the art that the present invention can be variously modified and changed without departing from the spirit and technical scope of the present invention described in claims described below. Therefore, the technical scope of the present invention should not be limited to the contents described in the detailed description of the specification, but should be defined by the claims.

### [INDUSTRIAL APPLICABILITY]

The present invention has industrial applicability in that it can quickly and accurately identify a wearing state even under unstable wearing situations, thereby eliminating unnecessary battery waste, and at the same time, provide various services such as exercise coaching services, sleep pattern analysis services, and the like.

## Claims

1. An optical-based skin detection sensor, comprising
a first light emitting unit that outputs first output light of a first wavelength range;
a second light emitting unit that outputs second output light of a second wavelength range different from the first wavelength range;
a light receiving unit that senses first reflected light and second reflected light reflected, respectively, from a predetermined object in which the first output light and the second output light are detected within a predetermined distance; and
a controller that determines whether the predetermined object is skin based on reflected light data for the first reflected light and the second reflected light sensed in the light receiving unit.

2. The optical-based skin detection sensor according to claim 1,
wherein the first output light is red light with a wavelength band of 400nm to 700nm, and
the second output light is infrared (IR) light with a wavelength of 850nm to 1050nm.

3. The optical-based skin detection sensor according to claim 1,
wherein the controller,
determines whether the predetermined object is skin in a stationary state based on the first reflected light and the second reflected light.

4. The optical-based skin detection sensor according to claim 3,
wherein the controller,
determines that the object is skin if a ratio of a normalized value of an intensity of the first reflected light and a normalized value of an intensity of the second reflected light falls within a predetermined criterion.

5. The optical-based skin detection sensor according to claim 1,
wherein the controller,
determines whether the predetermined object is skin in a moving state based on the first reflected light and the second reflected light.

6. The optical-based skin detection sensor according to claim 5,
wherein the controller,
determines that the object is skin if a ratio of a normalized value of a rate of change of intensity of the first reflected light and a normalized value of a rate of change of intensity of the second reflected light falls within a predetermined criterion.

7. The optical-based skin detection sensor according to claim 1,
wherein the controller,
controls the second light emitting unit in a non-wearing state to control it to output the second output light at a first output strength, and
detects a predetermined object within a predetermined distance based on the second reflected light for the second output light in the light receiving unit.

8. The optical-based skin detection sensor according to claim 7,
wherein the controller,
controls the first light emitting unit to output the first output light, when a predetermined object is detected within the predetermined distance.

9. The optical-based skin detection sensor according to claim 1,
wherein the reflected light data is data which consists of a combination of a signal by blood flow, P(t) and a signal by motion, M(t), and
includes signal patterns of the first and second reflected light.

10. The optical-based skin detection sensor according to claim 9,
wherein the controller,
filters only the P(t) by removing the M(t) included in the reflected light data based on a blind separation technique,
when the reflected light data includes the M(t) comprising a DC component equal to or greater than a predetermined criterion.

11. The optical-based skin detection sensor according to claim 10,
wherein the blind separation technique,
includes a PCA (Principal Component Analysis) method that minimizes cross-correlation of the reflected light data and an ICA (Independent Component Analysis) method that minimizes mutual information.

12. A wearable device comprising; a skin detection sensor comprising; a first light emitting unit that outputs first output light of a first wavelength range;
a second light emitting unit that outputs second output light of a second wavelength range different from the first wavelength range;
a light receiving unit that senses first reflected light and second reflected light reflected, respectively, from a predetermined object in which the first output light and the second output light are detected within a predetermined distance; and
a controller that determines whether the predetermined object is skin based on reflected light data for the first reflected light and the second reflected light sensed in the light receiving unit,
wherein a processor of the wearable device that controls the wearable device,
determines that it is in a wearing state upon determining that the type of the predetermined object is skin, and
controls activation of the wearable device based on the determined wearing state.

13. An optical-based skin detection method, which is a method of detecting optical-based skin by at least one processor of a wearable device, comprising
outputting first output light of a first wavelength range based on a first light emitting unit;
outputting second output light of a second wavelength range different from the first wavelength range based on a second light emitting unit;
sensing first reflected light and second reflected light reflected, respectively, from a predetermined object in which the first output light and the second output light are detected within a predetermined distance based on a light receiving unit;
determining whether the predetermined object is skin based on reflected light data for the sensed first reflected light and the second reflected light;
determining that it is in a wearing state upon determining that the type of the predetermined object is skin; and
controlling activation of the wearable device based on the determined wearing state.

14. The optical-based skin detection method according to claim 13,
wherein the determining whether the predetermined object is skin,
comprises determining whether the predetermined object is skin in a stationary state based on the first reflected light and the second reflected light, and
the determining whether the predetermining object is skin in a stationary state,
comprises determining that the object is skin if a ratio of a normalized value of an intensity of the first reflected light and a normalized value of an intensity of the second reflected light falls within a predetermined criterion.

15. The optical-based skin detection method according to claim 13,
wherein the determining whether the predetermined object is skin,
comprises determining whether the predetermined object is skin in a moving state based on the first reflected light and the second reflected light, and
the determining whether the predetermining object is skin in a moving state,
comprises determining that the object is skin if a ratio of a normalized value of a rate of change of intensity of the first reflected light and a normalized value of a rate of change of intensity of the second reflected light falls within a predetermined criterion.
